(19) Europäisches Patentamt

European Patent Office

Office européen des brevets

(11) **EP 4 708 299 A1**

(12) **EUROPEAN PATENT APPLICATION**

(43) Date of publication:
**11.03.2026  Bulletin 2026/11**

(21) Application number: **24306471.4**

(22) Date of filing: **09.09.2024**

(51) International Patent Classification (IPC):
**G16B 5/00** *(2019.01)*          **G16B 25/10** *(2019.01)*
**G16B 40/30** *(2019.01)*

(52) Cooperative Patent Classification (CPC):
**G16B 25/10; G16B 5/00; G16B 40/30**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Dassault Systèmes**
**78140 Vélizy-Villacoublay (FR)**

(72) Inventor: **El Hachem, Elie-Julien**
**Vélizy-Villacoublay (FR)**

(74) Representative: **Bandpay & Greuter**
**11 rue Christophe Colomb**
**75008 Paris (FR)**

(54)    **GENETIC DATA PROCESSING**

(57)    The disclosure notably relates to a computer-implemented method for genetic data processing. The processing method comprises obtaining (S10) a dataset comprising microbial gene expression data for a plurality of patients. The method comprises clustering (S20) the plurality of patients into a set of clusters based on the microbial gene expression data. The method comprises determining (S50) genes of which expression explains the clustering by selecting genes included in a set of metabolic pathways and/or by identifying genes exhibiting significantly different expressions across the clusters. The method forms an improved solution for patient stratification based on metagenomic data.

FIG. 1

EP 4 708 299 A1

**Description**

**TECHNICAL FIELD**

The disclosure relates to the field of computer programs and systems, and more specifically to a method, system and program for genetic data processing.

**BACKGROUND**

Metabolic diseases, including obesity, incur a significant economic burden estimated at $212.4 billion to $315.8 billion (in 2010), representing a 48.7 percent increase. Factors contributing to this rise include increased costs per obese individual, population growth, and a higher prevalence of obesity. Notably, not only has obesity surged, but metabolic conditions like atherosclerosis, liver cirrhosis, and type 2 diabetes have also seen an increase, underscoring the urgency of research in this domain as a public health priority.

One potential explanation for the increase in these diseases is the alteration of living conditions, which affects both individual health and their microbiome. Several studies have recognized the central role of intestinal microbiota in shaping the metabolic health of the host by producing various reactive components that could influence host metabolism.

In parallel, advancements in technologies such as metagenomics have enabled the scientific community to deepen its understanding of the connections between gut microbiota. Metagenomics is based on the analysis of genetic material, primarily DNA, extracted directly from environmental samples like fecal microbiota from human body. This approach provides a holistic view of entire microbial communities. Through high-throughput sequencing technologies such as next-generation sequencing (NGS), metagenomic studies generate vast amounts of sequence data, that can be analyzed using bioinformatic tools and software. Altogether, this methodology produces a vast amount of data, ranging from functional gene profiles (providing information about the functional potential of microbial communities by identifying and characterizing genes involved in various biological processes) to taxonomic composition (offering insights into the taxonomic composition of microbial communities by identifying the types and relative abundances of different micro-organisms present in the sample).

All these types of data possess inherent structures with differing scales, variability, and distributions, hindering simplistic analysis for meaningful information extraction. Adding to this complexity, human patients introduce significant variability, even within carefully selected cohorts, due to factors such as phenotypic variations (age, gender and/or previous conditions), dietary habits, and differential responses to treatments.

Current analyses typically address heterogeneity either through direct consideration (e.g., reducing the dimensionality) or by aggregating information into clusters (often arbitrary) for inter-patient comparisons. For example, several methodologies have been used such as Principal Component Analysis (PCA) and other developed to reduce the dimensionality in metagenomic studies such as Principal Coordinate Analysis (PCoA). PCoA centers its analysis on a distance matrix among samples, rather than the sample covariance matrix. Rather than directly analyzing the original observed data, PCoA opts to break down the distance matrix. Dimensionality reduction methods help visualize high-dimensional metagenomic data, aiding in the identification of distinct patient clusters. Techniques such as k-means clustering, hierarchical clustering, and self-organizing maps (SOM) are used to identify natural groupings within the data without prior labels. Alternatively, in the analysis of metagenomic data, variable selection methods like LASSO (Least Absolute Shrinkage and Selection Operator) have been employed.

These techniques effectively reduce the coefficients of less influential features to zero, thereby favoring the selection of highly correlated characteristics. However, a notable limitation when applied to metagenomic data is its inadequate handling of multicollinearity. Indeed, metagenomic datasets often comprise numerous features, such as genes, many of which exhibit high correlations due to functional redundancy or co-occurrence patterns.

More recently, some methodologies such as DeepMicro which is a deep learning framework that utilizes various autoencoders to generate robust low-dimensional representations from high-dimensional microbiome profiles for disease state prediction, have emerged. However, while these methods are developed with the aim for predicting the clinical status of patients mostly through hard clustering, they lack the capacity to stratify patients into transitory states, thereby substantially neglecting potential intermediate disease states in some individuals. Moreover, these methodologies fail to elucidate the shared characteristics among different patients within each group, showing a limitation in the term of information loss attributable to dimensionality reduction and multiple projections.

Within this context, there is still a need for an improved solution for genetic data processing.

**SUMMARY**

It is therefore provided a computer-implemented method for genetic data processing. This method is hereinafter referred to as the "processing method", or simply the "method". The processing method comprises obtaining a dataset comprising microbial gene expression data for a plurality of patients. The method comprises clustering the plurality of patients into a set of clusters based on the microbial gene expression data. The method comprises determining genes of which expression explains the clustering by selecting genes included in a set of metabolic pathways and/or by identifying genes exhibiting significantly different expressions across the clusters.

The processing method may comprise one or more of the following:

- The clustering comprises:

  ◦ Performing several iterations of a Latent Dirichlet allocation. Each iteration comprises assigning each patient to one of a plurality of topics;
  ◦ Determining a graph comprising nodes representing the plurality of patients and edges connecting pairs of nodes. The length of each edge connecting a pair of nodes is function of the number of occurrences of the patients represented by the connected nodes in the same topic over the several iterations of the latent Dirichlet allocation; and
  ◦ Performing a graph-based clustering of the determined graph;

- The graph is determined based on the Fruchterman-Reingold force-directed algorithm;
- The graph-based clustering is performed based on the Louvain clustering algorithm;
- The identifying of genes exhibiting significantly different expressions across the clusters comprises:

  ◦ Comparing the expression of each gene between each cluster; and
  ◦ Retaining genes with significantly different expressions between the clusters based on the results of the comparison;

- The comparison of the gene expression is performed based on a Kruskal-Wallis test computing a respective p-value for each gene, the retained genes having optionally p-values lower than 1 %;
- Selecting the set of metabolic pathways by extracting, from a database of existing metabolic pathways, the most significantly enriched metabolic pathways in terms of genes;
- The selecting of the set of metabolic pathways is performed based on a Fisher's exact test computing a respective p-value for each metabolic pathway. The selected metabolic pathways of the set have optionally p-values lower than 5 %; and/or
- Filtering, from a database of genes, genes of which relative expression is above a threshold. The genes are determined from the genes remaining after the filtering.

It is further provided a computer-implemented method of use of the clustering. This method is hereinafter referred to as the "using method". The using method comprises obtaining microbial gene expression data for a patient. The microbial gene expression data include the expression of the genes determined in the processing method. The using method comprises determining the cluster to which the patient belongs based on the expression of the determined genes for the patient.

The using method may comprise one or more of the following:

- Determining a closest patient among the patients of the determined cluster; and/or
- The closest patient is determined using the Bray-Curtis distance.

It is further provided a computer program comprising instructions for performing the processing method and/or the using method.

It is further provided a computer readable storage medium having recorded thereon the computer program.

It is further provided a system comprising a processor coupled to a memory, the memory having recorded thereon the computer program. The processor may optionally be coupled to a graphical user interface.

It is further provided a device comprising a data storage medium having recorded thereon the computer program.

The device may form or serve as a non-transitory computer-readable medium, for example on a SaaS (Software as a service) or other server, or a cloud based platform, or the like. The device may alternatively comprise a processor coupled to the data storage medium. The device may thus form a computer system in whole or in part (e.g. the device is a subsystem of the overall system). The system may further comprise a graphical user interface coupled to the processor.

## BRIEF DESCRIPTION OF THE DRAWINGS

Non-limiting examples will now be described in reference to the accompanying drawings, where:

- FIGs. 1 and 2 show flowcharts of examples of the method;
- FIG. 3 shows an example of the determined graph;
- FIG. 4 illustrates an example of distance-based analysis of the clusters;
- FIG. 5 shows an example of a comparison between results obtained using the processing method and a random forest; and
- FIG. 6 shows an example of the system.

**DETAILED DESCRIPTION**

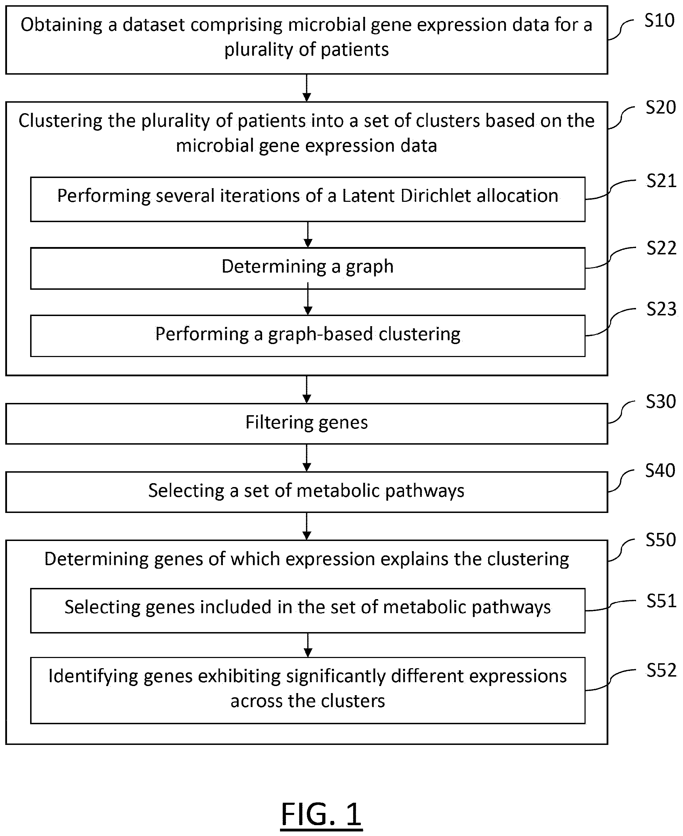

With reference to the flowchart of FIG. 1, it is proposed a computer-implemented method for genetic data processing. This method is hereinafter referred to as the "processing method", or simply the "method". The processing method comprises obtaining a dataset comprising microbial gene expression data for a plurality of patients. The method comprises clustering the plurality of patients into a set of clusters based on the microbial gene expression data. The method comprises determining genes of which expression explains the clustering by selecting genes included in a set of metabolic pathways and/or by identifying genes exhibiting significantly different expressions across the clusters. The method forms an improved solution for genetic data processing.

Notably, one of the main advantages is that the method firstly enables the plurality of patients to be clustered based on their microbial gene expression data, and thus to identify groups of patients with similarities in microbial gene expression. Moreover, the method allows efficiently to determine genes of which expression explains the clustering. Indeed, the method enables to isolate the genes significantly expressed between the groups, and thus to verify that these genes indeed facilitate the stratification of patients within these clusters. This is done, for example, by comparing the enriched genes for each metabolic pathway among the previously defined clusters.

Furthermore, the method enables either rediscovering the stratification or solely assigning a new patient to a cluster of individuals. Indeed, the method allows determining the genes of which expression explains the clustering, and thus, on the basis of the expression of these genes only for a patient, to find the cluster of this patient. The method therefore allows selecting intestinal microbial genes for patient stratification into various homogeneous subgroups.

The method is computer-implemented. This means that steps (or substantially all the steps) of the method are executed by at least one computer, or any system alike. Thus, steps of the method are performed by the computer, possibly fully automatically, or, semi-automatically. In examples, the triggering of at least some of the steps of the method may be performed through user-computer interaction. The level of user-computer interaction required may depend on the level of automatism foreseen and put in balance with the need to implement user's wishes. In examples, this level may be user-defined and/or pre-defined.

A typical example of computer-implementation of a method is to perform the method with a system adapted for this purpose. The system may comprise a processor coupled to a memory and a graphical user interface (GUI), the memory having recorded thereon a computer program comprising instructions for performing the method. The memory may also store a database. The memory is any hardware adapted for such storage, possibly comprising several physical distinct parts (e.g. one for the program, and possibly one for the database).

The dataset comprising the microbial gene expression data, the resulting set of clusters and/or the determined genes of which expression explains the clustering may be stored in a database. By "database", it is meant any collection of data (i.e. information) organized for search and retrieval (e.g. a relational database, e.g. based on a predetermined structured language, e.g. SQL). When stored on a memory, the database allows a rapid search and retrieval by a computer. Databases are indeed structured to facilitate storage, retrieval, modification, and deletion of data in conjunction with various data-processing operations. The database may consist of a file or set of files that can be broken down into records, each of which consists of one or more fields. Fields are the basic units of data storage. Users may retrieve data primarily through queries. Using keywords and sorting commands, users can rapidly search, rearrange, group, and select the field in many records to retrieve or create reports on particular aggregates of data according to the rules of the database management system being used.

The method is for genetic data processing, including the processing of microbial gene expression data. This involves analyzing and interpreting information found in genetic data, such as DNA sequences, mutations, genetic variations, and other elements related to genes and genomes. Genetic data processing may be used in genomic research, personalized medicine, medical genetics, and various other scientific and medical disciplines. For example, the using method may be included in a medical process, which may comprise, after performing the using method, using the determined cluster of the patient for assisting a medical practitioner (such as a doctor or a nurse) to treat and/or cure a patient disease and/or infection. Specifically, the using method may comprise comparing the patient's information with data from other patients within the same cluster.

For example, the using method may comprise determining the closest patient from among all the other patients that are included in the determined cluster (the patient within the cluster who most closely resembles the patient under study from a genetic point of view), and deducing information about the patient under study by similarity with the determined closest patient. By analyzing the similarities with the closest patient, the using method may comprise inferring additional information about the patient under study, such as likely disease progression, response to treatments, or potential risk factors. This comparative approach allows for a more personalized and informed treatment plan by leveraging the medical histories and outcomes of similar patients. For instance, if the closest patient in the cluster responded well to a particular therapy, the same treatment might be considered for the patient under study. The using method allows finding and suggesting such information to the medical practitioner to assist them in their practice.

The distance between patients within a same cluster may be calculated in different ways. For example, it may be determined on the basis of each patient's metagenomic data. Alternatively, if clinical data are available, it may also be calculated from the clinical data. As a general rule, the Euclidean distance may be used, but the Minkowski distance may also be used. The method may also use the graph and the resulting clustering to calculate a geodesic distance (the shortest distance in terms of number of edges between two nodes).

In example, the closest patient may be determined using the Bray-Curtis distance. The Bray-Curtis distance may be calculated using the formula:

$$ d_{BC}(p, q) = \frac{\sum_{i=1}^{m} \left| x_{i,p} - x_{i,q} \right|}{\sum_{i=1}^{m} \left( x_{i,p} + x_{i,q} \right)} $$

wherein, $p$ and $q$ are a pair of patients, m is the number of genes, $x_{i,p}$ and $x_{i,q}$ the value of gene $i$ for patient $p$ and patient $q$ respectively.

The using method may then comprise outputting information about the determined closest patient. Each other patient in the cluster may be suffering from the same disease and/or infection, and the outputted information may indicate a stage of disease and/or infection for the closest one or more patients, a treatment given to them, and/or a response to that treatment. The outputted information may be used to infer similar information about the patient (e.g., a stage of disease and/or infection and/or to predict a response to a given treatment). By allowing the patient to be compared only with a subset of patients in the database (i.e., with patients included in the same cluster), the method improves the inference of patient information from the database. In particular, as the clusters are based on microbial gene expression data, the method enables the patient to be compared with patients sharing the same microbial gene expression, allowing this to be taken into account during inference.

Moreover, because the method improves the deduction of patient information from the patient database, it also improves assisting to the medical practitioner to treat and/or cure the patient, and thus ultimately the treatment of the patient's disease and/or infection. In particular, the using method allows targeting genes of which expression has an influence on the result. Indeed, the microbial gene expression data obtained for the patient may include only the expression of the genes identified as exhibiting significantly different expressions across the clusters. Thus, the using method also allows reducing the number and complexity of genes to be searched in the patient data.

The obtaining S10 of the dataset is now discussed.

The dataset comprises microbial gene expression data for the plurality of patients. For example, the microbial gene expression data may comprise, for each patient of the plurality, information on the expression of microbial genes for the patient. The expression of a gene may refer to information about the process by which the information contained in the gene is used to produce a functional product, usually a protein, but sometimes a non-coding RNA. This process may be fundamental for the functioning of cells and the manifestation of genetic traits. The microbial gene expression data may be extracted from environmental samples like fecal microbiota from human body. The microbial gene expression data for the plurality of patients in fecal matter may characterize the intestinal microbiota of these patients, i.e., all the micro-organisms living in the intestines of the plurality of patients. The microbial gene expression data allows identifying, for each patient, the bacterial species present in the intestinal microbiota of the patient, their relative abundance, and the functions they can perform.

The obtaining S10 may comprise determining the dataset comprising the microbial gene expression data for the plurality of patients. The determining of the dataset comprising the microbial gene expression for the plurality of patients may be performed in any manner. For example, the determining of the dataset may comprise extracting, from real environmental samples (e.g., like fecal microbiota from human body), the microbial gene expression data for the plurality of patient. Alternatively, the microbial gene expression data may already have been extracted by the time the method is executed. For example, the microbial gene expression data may have been recorded in a memory. In that case, the obtaining S10 may comprise extracting, from this memory, the recorded microbial gene expression data for the plurality of patients. Alternatively, yet, the memory may store microbial gene expression data only for a portion of the plurality of patients. In that case, the obtaining S10 may comprise the extracting, from the memory, of the microbial gene expression data for this portion of the patients, and extracting, from real environmental samples, the microbial gene expression data for the remaining patients.

The clustering S20 of the plurality of patients is now discussed.

The clustering S20 of the plurality of patients may be performed in any manner. For example, the clustering S20 of the plurality of patients may be performed based on any known algorithm. The clustering S20 may comprise determining the set of clusters and assigning each patient to one of the clusters of the set. The clustering S20 may consider the microbial gene expression data of the patients to form the clusters and may group together patients with similar microbial gene expression data within the same cluster. The number of clusters may be predetermined, or alternatively, it may be inferred during the clustering S20. This inference may be aimed at optimizing one or more criteria, such as maximizing the similarity of microbial gene expression data within the same cluster, maximizing the distance between patients in different clusters, and/or minimizing the total number of resulting clusters.

In examples, the clustering process S20 may comprise three steps: performing S21 several iterations of a Latent Dirichlet allocation, determining S22 a graph, and performing S23 a graph-based clustering. Performing the clustering S20 based on these three steps enhances the clustering of the patients, as it allows for maximizing the similarity of microbial gene expression data within the same cluster, maximizing the distance between patients in different clusters, and minimizing the total number of resulting clusters. Each of these three steps, S21, S22, and S23, is discussed in more detail in the following paragraphs.

The first step S21 consists in performing the several iterations of the Latent Dirichlet allocation (each iteration resulting in a respective Latent Dirichlet allocation). A Latent Dirichlet allocation is a process which consists in assigning, for a given plurality of topics, each patient to one of the given plurality of topics. Each iteration of the Latent Dirichlet allocation provides a respective assignment of the patients in the given plurality of topics. The several iterations may provide different assignments of the plurality of patients (in terms of the number of topics considered at each iteration and in terms of the resulting distribution of patients within the topics considered at each iteration). The number of topics may for example be a hyperparameter optimized, e.g., using Grid Search, enabling the best desired performance to be obtained. For example, the method may consider an interval for the number of topics ranging from 3 (being the minimum number of potential topics) to $n$ potential topics ($n$ being a positive integer).

Then, the second step S22 consists in determining a graph representing the different assignments resulting from the several iterations of the Latent Dirichlet allocation. The determining of the graph may comprise counting, for each pair of patients, the number of times the two patients in the pair are found in the same topic during the different iterations of the latent Dirichlet allocation (each time the two patients are found in the same topic being an occurrence of the two patients in the same topic). The counts obtained for each pair of patients may be represented in a matrix. Then, the determining of the graph may comprise arranging the nodes representing the patients so that the distance between each pair of nodes (i.e., the length of the edge between the two nodes) is approximately proportional to the counted number of occurrences for those two nodes. For example, the determining of the graph may comprise moving the nodes to minimize a cost that weights the deviation from this proportionality between the length of each edge and the counted number of occurrences for the nodes it connects.

The determining of the graph based on the counts may be performed based on a known algorithm, such as the Fruchterman-Reingold force-directed algorithm. For example, the determining of the graph may comprise an initial step of randomly projecting all nodes into a space, without overlapping. The algorithm may treat nodes as repelling objects, while the edges may be considered as springs that keep the nodes connected. Then, the determining of the graph may execute an iterative process which comprises, at each iteration, calculating the attractive and repulsive forces between each pair of nodes and adjusting their positions accordingly until convergence is achieved (the said cost weighting the deviation from the proportionality being minimized), meaning the nodes no longer move within the space. For graphic purposes, when the graph is displayed, the thickness of each edge may also depend on the number of occurrences (the shorter and thicker the line, the stronger the number of occurrences).

Then, the third step S23 consists in performing a graph-based clustering of the determined graph. The graph-based clustering may be performed based on an algorithm configured for dividing the graph into sub-communities of nodes (i.e., the sub-communities corresponding to the resulting clusters) to maximize modularity. The algorithm may take as input the computed matrix representing the counts obtained for each pair of patients. The modularity may quantify a density of edges within communities versus edges between different communities. The algorithm may for example be the Louvain clustering algorithm, or the Leiden clustering algorithm. The algorithm may execute the following steps. A first step may comprise randomly assigning each node to a community. A second step may comprise moving nodes within each community to optimize modularity (if modularity increases after moving a node, then the algorithm may allocate it to a new community). A third step may comprise merging neighboring communities (to create larger, more homogeneous communities). A fourth step may be a refining step The refining step may comprise moving nodes between newly merged communities and calculating modularity (for optimizing again modularity).

The resulting clustering may be used in a number of ways. For example, one may be to stratify patients from metagenomic data. However, it can also be used to stratify patients from other types of data that follow a probability distribution, such as a Dirichlet distribution.

The determining S50 of genes of which expression explains the clustering is now discussed. The genes of which expression explains the clustering are hereinafter referred to as the "explanatory genes".

The determining S50 of the explanatory genes comprises the identifying S52 of genes exhibiting significantly different expressions across the clusters. The genes identified during this step S52 are the determined explanatory genes. In step S52, the genes may be identified from a database referencing genes, genomes and/or biological pathways, for example a known database such as the KEGG (Kyoto Encyclopedia of Genes and Genomes) database. In example, the determining S50 of the explanatory genes may also comprise, prior to this identifying step S52, the selecting S51 of the genes included in the set of metabolic pathways. In that case, the genes are identified in step S52 from the genes selected in step S51 (i.e., only from these). The genes may be identified in step S52 from all the genes referenced in the said (e.g., known) database. These two steps S51 and S52 are now discussed in more detail in the following paragraphs.

At step S51, the genes may be selected from all the genes referenced in the said (e.g., known) database. For example, the database may list all metabolic pathways (including those within the said set of metabolic pathways) and may comprise information indicating, for each gene, which metabolic pathway(s) the gene is part of. The selection S51 of the genes included in the set of metabolic pathways may be based on this information and may comprise extracting genes indicated as included in at least one of the metabolic pathways of the set in the said information. For example, the selection S51 may comprise scanning all genes of the database and retaining only genes indicated as being involved in at least one of the metabolic pathways of the set.

The identifying S52 of the genes exhibiting significantly different expressions across the clusters may comprise the following two steps. In a first step, the identifying S52 may comprise comparing the expression of each gene between each cluster. This comparison of the gene expression may be performed based on a Kruskal-Wallis test, which is a non-parametric test, based on rank assignment, identifying significant differences in terms of gene expression between the clusters. The Kruskal-Wallis test is particular adapted for comparing gene expression between clusters because the data do not follow known distributions (such as Gaussian types). The Kruskal-Wallis test may comprise the following steps. A first step comprises combining and arranging patient metagenomic data in ascending order. A second step comprises assigning ranks to the data. A third step comprises calculating a Kruskal-wallis statistic. A fourth step comprises comparing the calculated Kruskal-wallis statistic with a Chi-square distribution, thereby determining a respective p-value. The steps may be repeated as many times as there are genes in the dataset, thereby computing a respective p-value for each gene.

In a second step, the identifying S52 may comprise retaining genes with significantly different expressions between the clusters based on the results of the comparison. For example, the comparison of the gene expression may compute a respective p-value for each gene, and the method may retain only genes having a respective p-value lower than a threshold (e.g., 1%). The identified explanatory genes may be the genes retained during this step.

A metabolic pathway is a linked series of chemical reactions occurring within a cell. The set of metabolic pathways considered by the method may represent a portion of all known metabolic pathways (e.g., referenced in the said database).

In examples, the method may comprise a step of selecting S40 the set of metabolic pathways. The selection S40 of the set of metabolic pathways may depend on the disease or infection being studied. The selection S40 is performed by extracting from a database of existing metabolic pathways, the most significantly enriched metabolic pathways in terms of genes. Metabolic pathways may be referenced in a known database (e.g., KEGG). In such database, each metabolic pathway may be associated with a list of genes or a list of metabolites. However, a gene may be associated with several different pathways. A metabolic pathway is said to be enriched when it has several genes in it.

The selection S40 of the set of metabolic pathways may performed by conducting an enrichment analysis of metabolic pathways to detect various enriched pathways, e.g., among all metabolic pathways referenced in the database. The selected set of metabolic pathways may comprise the most significantly enriched pathways in terms of genes according to this conducted enrichment analysis. For example, the selected set may comprise a predetermined number of the most enriched pathways or may comprise metabolic pathways having an enrichment higher than a threshold. The selection S40 of the set of metabolic pathways may be performed based the enrichment p-value result of the Fisher's exact test which may be used for computing a respective p-value for each metabolic pathway. The selected metabolic pathways of the set may be the one having p-values lower than a threshold (e.g., 5 %).

In examples, the method may comprise the filtering S30 of the genes. The step of filtering S30 may be executed prior to the selection of the set of metabolic pathways (step S40) and the determination of the explanatory genes (step S50). In that case, the explanatory genes may be determined from the genes remaining after the filtering. For example, the filtering S30 may comprise attributing a value to each gene initially referenced in the database, and the filtering S30 may comprise retaining only genes having a value higher than a threshold. This value may represent a relative expression of the gene (i.e., its normalized expression). The relative expression of a gene (or relative abundance) may be calculated as in the paper of Jie et al, 2017, "The gut microbiome in atherosclerotic cardiovascular disease", Nat Commun 8, 845, https://doi.org/10.1038/s41467-017-00900-1. In a first step, the data may be sequenced, and then the various reads obtained may be aligned with a reference catalog containing gene sequences (gene counting). One of the identification criteria here may include an alignment greater than 35% and a score greater than 60. In a second step, the relative abundance of a gene may be then calculated by dividing the raw abundance of that gene by the total abundance of all reads in the sample. In a third step, the relative abundance of a KO may be calculated by dividing the raw abundance of that KO by the total abundance of all genes in the sample. This value, which is an hyperparameter, may have been measured at the time of the extraction of the gene from environmental samples. In that case, the threshold used for filtering may be less than 1E-3 and/or greater than 1E-5, for example it may be substantially equal to 5E-4. The filtering S30 improves the efficiency and accuracy of data processing and the detection of genes of significant importance. Indeed, the filtering S30 enables the algorithm to focus only on a subset of relevant genes from the database.

When the method comprises the filtering step S30, the genes may be identified in step S52 from the genes remaining after the filtering (i.e., genes having a value higher than the threshold). When the method comprises both the filtering step S30 and the selection step S51, the genes may be selected in step S51 from the genes remaining after the filtering (i.e., genes having a value higher than the threshold).

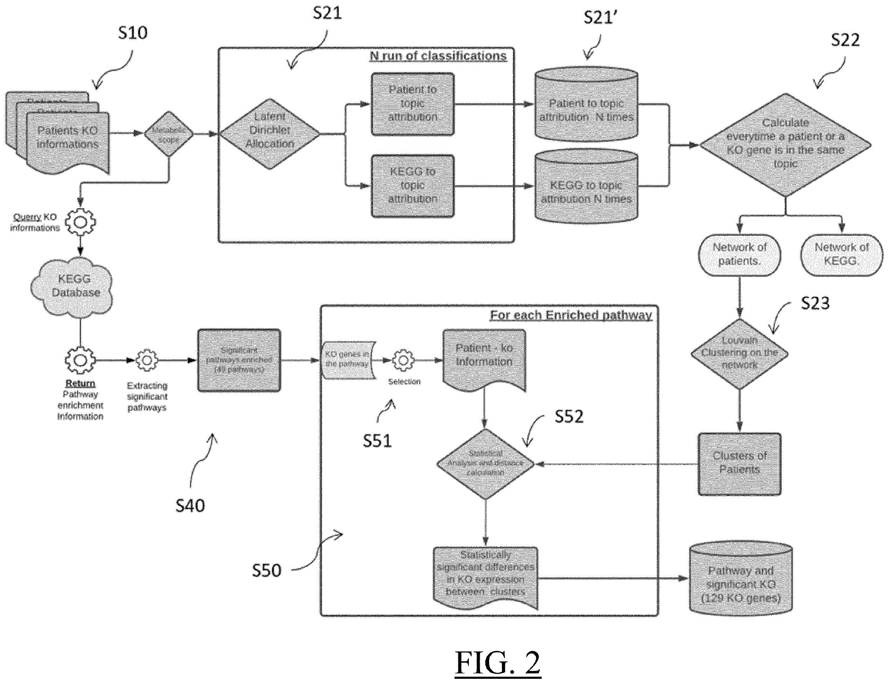

With reference to FIGs. 2 to 6, examples of implementations of the method are now presented.

Cardiometabolic display a significant global health challenge, necessitating a deeper understanding of disease progression and patient trajectories. The advent of high-throughput technologies has generated a huge amount of heterogeneous and complex data, reflecting the unique characteristics of each patient. To effectively extract meaningful insights from this vast repository of information, the method may be executed in order to stratify them. Patient stratification is a process that group population of patients into clinically or molecularly defined subgroups based on identifiable characteristics that are associated with disease course, prognosis, or therapeutic response.

One of the key challenges addressed by the method is to effectively stratify patients within the context of metabolic diseases, using a high-dimensional dataset (metagenomics), and subsequently reducing dimensionality to extract valuable insights. Especially, the method implements a computational framework that initially stratifies individuals into homogeneous sub-populations. Subsequently, it identifies microbial genes exhibiting significantly different levels of expression across these groups, making the assumption that these metagenomic genes can serve as biomarkers. To validate this hypothesis, the method may comprise applying a classification-based algorithm to the data and successfully reclassified individuals into their respective groups. Thus, the utilization of previously identified genes can be used as biomarkers for stratification.

The implementation of the method is centered on analyzing data related to metagenomics in patients with cardiovascular diseases. It demonstrates the ability to stratify patients based on their intestinal microbiota sequencing data into distinct groups, cluster them and identify specific bacterial genes that differentiate between them and could serve as biomarkers for stratification. Following an examination of metabolic pathway enrichment across the dataset, the method may comprise conducting statistical assessments on the expression of metagenomic genes for each enriched pathway between each patient cluster. Subsequently, these genes may be used in a patient classification task, wherein a notably high proficiency in accurately classifying patients into previously defined clusters compared to clinical phenotype categorization may be observed. Hence, the method may be used for effectively stratifying individuals and yields a set of genes suitable for utilization as biomarkers for potential therapy and disease classification state.

"Omics" refers to the process of capturing the entirety of various biological molecules (DNA, RNA, proteins, etc.) within a cell, organism, or population. They are large-scale datasets presenting various technological challenges, the primary one being the extraction of information that holds both mathematical relevance and biological meaning. One of the key technological problems solved by the method is stratifying patients into biologically homogeneous groups and identifying clusters of patients sharing similarities in the data, that could reflect more accurately patient state compared to clinical state that are defined based on clinical biomarkers. Subsequently, it is necessary to understand the distinctions among patients sharing the same "omics" leading to this stratification and identify potential discriminant biomarkers between the groups, which could be potentially actionable and targetable in a therapeutic strategy.

The method solves this problem by implementing a framework that combines patient stratification, identification of enriched signaling pathways (pathway analysis), and a methodology for selecting discriminatory bacterial genes among patient groups established by the stratification. Furthermore, the method may further comprise using these genes to classify certain patients and demonstrate that they enable classification of patients into subgroups previously established by stratification with higher accuracy than clinical groups.

One of the main advantages of the method is that it initially allows the use of existing methodologies to stratify patients into a network. Then, by applying a clustering method to this network, the method allows identifying groups of patients with similarities. Finally, by comparing the enriched genes for each pathway among the previously defined clusters, the method allows isolating the genes significantly expressed between the groups and verifying that these genes indeed facilitate the stratification of patients within these clusters.

Thus, by using only this gene pool, it is possible to either rediscover the stratification or solely assign a new patient to a cluster of individuals. In conclusion, the method enables the selection of intestinal microbiota genes for patient stratification into various homogeneous subgroups.

Some terms are now explained. KEGG (Kyoto Encyclopedia of Genes and Genomes) is a collection of databases that store information about genes, genomes, biological pathways. KO (KEGG Ortholog) is a group of genes that are considered to be functionally equivalent across different organisms. This means that the genes in a KO group all carry out the same basic function, even though they may have different DNA sequences.

The method allows identifying specific biomarkers enabling patient stratification. A biomarker is defined as a measurable indicator of a biological state or condition to diagnose, to monitor health conditions, or assess responses to treatments. In a first step, using available data from a database referencing a first set of genes (e.g., a set of 7125 KEGG Orthologue genes), the method comprises filtering S30 genes of which relative expression is above a threshold (e.g., 5E-4). The filtering step S30 may comprise applying a metabolic scope defined as a threshold (values below this threshold can be attributed to noise caused by experimental conditions or detection limitations). This threshold may be set at 5E-4, thereby excluding all genes whose relative expression is lower than this value. Thus, the filtering S30 allows obtaining a second set of genes which is a subset of the first set of genes (e.g., a subset of 922 genes), lower than the first number, that can be used and analyzed in the stratification illustrated in FIG. 2.

Then, in step S20, the method comprises applying a Latent Dirichlet Allocation S21, allowing to simultaneously assign each patient a probability of belonging to a topic, and each gene a probability of belonging to a topic as well. At the end of the various allocation runs, the method comprises allocating S21' both patient and gene to the topic with the highest probability. The process is iterated multiple times, allowing to calculate S22 the number of times each patient is associated with another within the same topic, and subsequently represent the patients in the form of a graph (using a display based on the Fruchterman-Reingold force-directed algorithm).

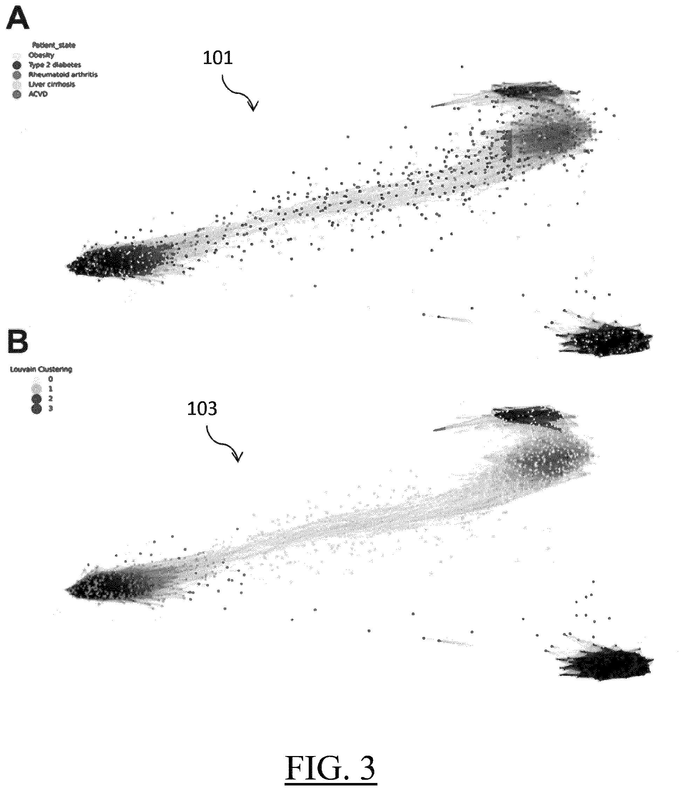

FIG. 3 illustrates two graphical representations of the determined graph of patients stratified based on metagenomics: a first representation 101 wherein patients are highlighted based on their clinical states and a second representation 103 wherein patients are highlighted based on their clustering attribution (Louvain Clusters). The resulting matrix is then clustered S23 using an algorithm called the Louvain Clustering based on community detection. Patients are thus clustered into subgroups, homogeneous in terms of metagenomic gene expression (see the example of resulting graph in FIG. 3).

In addition to this, alongside the second set of 922 gene KOs, the method comprises conducting S40 an enrichment analysis of metabolic pathways to detect various enriched pathways. Pathway enrichment is a computational process that identifies biological functions statistically overrepresented in a set of genes compared to what would be expected by chance, as explained in the paper of Chicco, D. and Agapito, G., 2022, "Nine quick tips for pathway enrichment analysis", PLoS Comput Biol 18, e1010348, https://doi.org/10.1371/journal.pcbi.1010348.

Out of the 922 genes, 515 genes may be recognized as being included in existing metabolic pathways in the KEGG database (see FIG. 2), thereby obtaining a third set of 515 genes. The method comprises extracting these metabolic pathways and selecting S40 only the most significantly enriched metabolic pathways in terms of genes (adjusted p-value < 0.05, p-value quantifying the strength of evidence against the null hypothesis by reflecting the probability of observing such extreme results, assuming the null hypothesis is true). In this example, this yields to a set of 49 significantly enriched metabolic pathways (see Table 1 below).

Table 1: Pathways significantly enriched (p-value adjusted < 0.05)

| | | Pathway Description | GeneRatio | pvalue | p.adjust |
|---|---|---|---|---|---|
| | ko01230 | Biosynthesis of amino acids | 55/515 | 7.81E-29 | 1.47E-26 |
| | ko03010 | Ribosome | 37/515 | 1.35E-21 | 1.27E-19 |
| | ko00500 | Starch and sucrose metabolism | 29/515 | 6.62E-18 | 4.15E-16 |
| | ko00520 | Amino sugar and nucleotide sugar metabolism | 29/515 | 3.78E-13 | 1.77E-11 |
| | ko01240 | Biosynthesis of cofactors | 46/515 | 4.76E-13 | 1.79E-11 |
| | ko02060 | Phosphotransferase system (PTS) | 18/515 | 6.90E-11 | 2.16E-09 |
| | ko00010 | Glycolysis / Gluconeogenesis | 21/515 | 2.43E-10 | 6.52E-09 |
| | ko00052 | Galactose metabolism | 18/515 | 2.89E-10 | 6.78E-09 |
| | ko01232 | Nucleotide metabolism | 22/515 | 5.91E-10 | 1.20E-08 |
| | ko01200 | Carbon metabolism | 40/515 | 6.39E-10 | 1.20E-08 |
| | ko00620 | Pyruvate metabolism | 22/515 | 2.75E-09 | 4.70E-08 |
| | ko00521 | Streptomycin biosynthesis | 9/515 | 2.19E-08 | 3.43E-07 |
| | ko00051 | Fructose and mannose metabolism | 19/515 | 2.95E-08 | 4.26E-07 |
| | ko00230 | Purine metabolism | 27/515 | 4.32E-08 | 5.80E-07 |
| | ko00240 | Pyrimidine metabolism | 18/515 | 1.21E-07 | 1.52E-06 |
| | ko00250 | Alanine, aspartate and glutamate metabolism | 13/515 | 1.28E-06 | 1.51E-05 |

(continued)

| | | Pathway Description | GeneRatio | pvalue | p.adjust |
|---|---|---|---|---|---|
| | ko01210 | 2-Oxocarboxylic acid metabolism | 16/515 | 2.71E-06 | 2.99E-05 |
| | ko01250 | Biosynthesis of nucleotide sugars | 23/515 | 2.98E-06 | 2.99E-05 |
| | ko00270 | Cysteine and methionine metabolism | 17/515 | 3.02E-06 | 2.99E-05 |
| | ko00030 | Pentose phosphate pathway | 14/515 | 3.99E-06 | 3.75E-05 |
| | ko03440 | Homologous recombination | 12/515 | 1.06E-05 | 9.48E-05 |
| | ko04112 | Cell cycle - Caulobacter | 8/515 | 1.15E-05 | 9.80E-05 |
| | ko00710 | Carbon fixation in photosynthetic organisms | 8/515 | 2.39E-05 | 0.000195012 |
| | ko03430 | Mismatch repair | 9/515 | 2.49E-05 | 0.000195012 |
| | ko00770 | Pantothenate and CoA biosynthesis | 9/515 | 3.01E-05 | 0.000226513 |
| | ko00730 | Thiamine metabolism | 8/515 | 3.74E-05 | 0.000270589 |
| | ko00290 | Valine, leucine and isoleucine biosynthesis | 6/515 | 4.23E-05 | 0.000294268 |
| | ko00541 | O-Antigen nucleotide sugar biosynthesis | 13/515 | 6.35E-05 | 0.000426049 |
| | ko00650 | Butanoate metabolism | 14/515 | 7.08E-05 | 0.000459012 |
| | ko00040 | Pentose and glucuronate interconversions | 12/515 | 0.000103922 | 0.000651244 |
| | ko00550 | Peptidoglycan biosynthesis | 9/515 | 0.000116124 | 0.000704239 |
| | ko00983 | Drug metabolism - other enzymes | 7/515 | 0.000128478 | 0.000754408 |
| | ko00970 | Aminoacyl-tRNA biosynthesis | 10/515 | 0.000132423 | 0.000754408 |
| | ko00020 | Citrate cycle (TCA cycle) | 10/515 | 0.000150644 | 0.000832974 |
| | ko03030 | DNA replication | 9/515 | 0.000308694 | 0.001656879 |
| | ko00300 | Lysine biosynthesis | 8/515 | 0.000317275 | 0.001656879 |
| | ko00061 | Fatty acid biosynthesis | 7/515 | 0.000470356 | 0.002389917 |
| | ko00220 | Arginine biosynthesis | 8/515 | 0.001465797 | 0.007251839 |
| | ko00780 | Biotin metabolism | 5/515 | 0.001539372 | 0.007420562 |
| | ko02024 | Quorum sensing | 21/515 | 0.001745512 | 0.008203905 |
| | ko04980 | Cobalamin transport and metabolism | 4/515 | 0.002890823 | 0.01325548 |
| | ko00790 | Folate biosynthesis | 9/515 | 0.003546715 | 0.015506566 |
| | ko01212 | Fatty acid metabolism | 9/515 | 0.003546715 | 0.015506566 |
| | ko00670 | One carbon pool by folate | 6/515 | 0.004511993 | 0.018955303 |
| | ko00720 | Carbon fixation pathways in prokaryotes | 11/515 | 0.004537174 | 0.018955303 |
| | ko00450 | Selenocompound metabolism | 5/515 | 0.006558707 | 0.026805149 |
| | ko00195 | Photosynthesis | 7/515 | 0.007967224 | 0.031868898 |
| | ko00640 | Propanoate metabolism | 9/515 | 0.009087039 | 0.035590904 |
| | ko00460 | Cyanoamino acid metabolism | 5/515 | 0.009525181 | 0.036545592 |

For each of these enriched pathways, the method may comprise extracting S51 all the genes included in them and comparing the expression of each gene between each cluster previously established. The method then comprises retaining only the genes with significantly different expression between the clusters (adjusted p-values < 0.01, using a Kruskal-Wallis test).

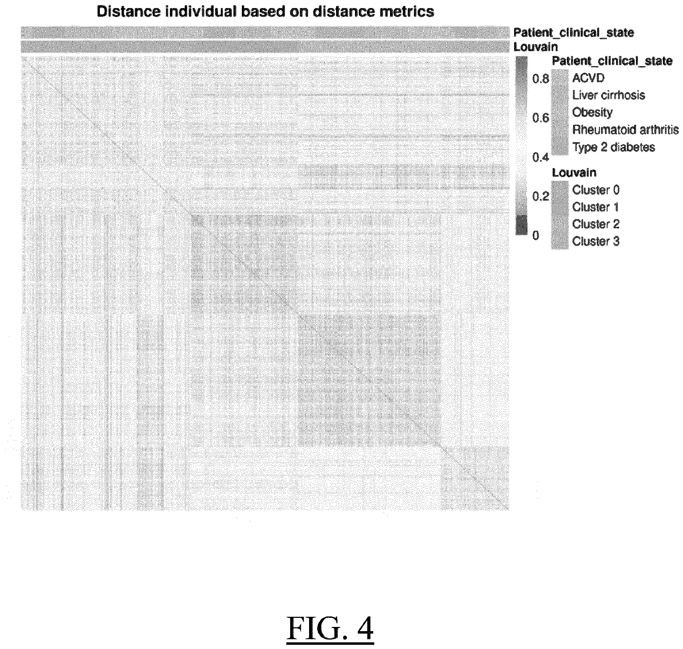

In this example of implementation, the method then comprises computing the Bray-Curtis distance for all patients to assess the used hypothesis. There is a clustering tendency among patients within each cluster. Notably, organizing patients according to their cluster assignation reveals a consistent pattern. FIG. 4 illustrates this distance-based analysis using the Bray-Curtis metric on the set of 129 genes identified and significantly expressed. Individuals within the same cluster have a low distance between them (indicating closeness), thus exhibiting similarity in their data). Therefore, patients sharing the same cluster exhibit similarity at least in their metagenomic characteristics, irrespective of their clinical phenotype.

The utility of the identified genes in discriminating between individuals is demonstrated by employing a Random Forest approach utilizing the previously identified set of genes as input. The predictive output encompasses either the patient's assigned cluster, determined by prior stratification, or the individual's clinical classification.

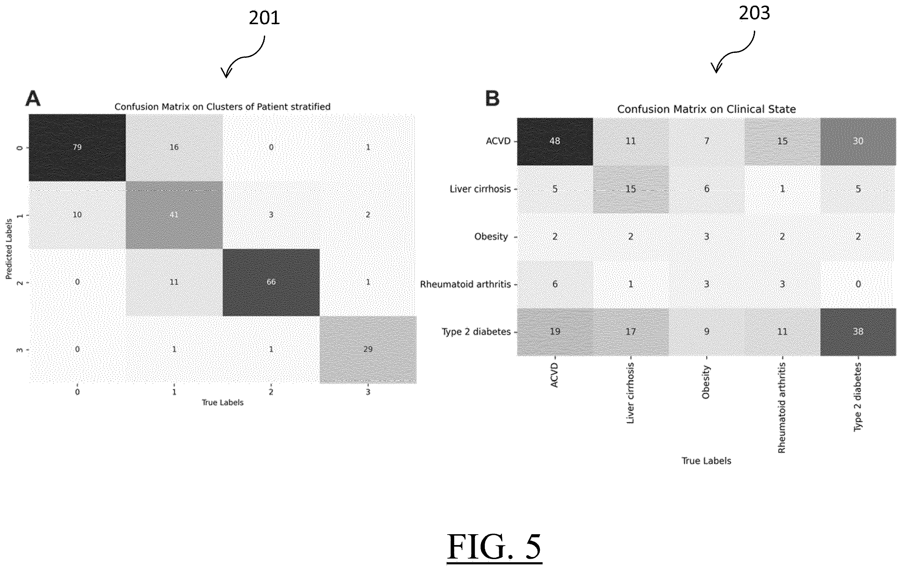

The dataset is partitioned into training and testing subsets, with an 80% allocation for training and 20% for testing. Subsequently, the Random Forest model parameters are fine-tuned. There is a high level of accuracy in classifying patients within each cluster (Mean accuracy across 20 iterations: 0.86). A visual representation of the classification performance, in the form of a confusion matrix 201 of predicted patient clusters versus actual patient clusters, is presented in FIG. 5.

Concurrently, there is a decreased accuracy when classifying patients into distinct clinical phenotype groups (Mean accuracy across 20 iterations: 0.43). Another confusion matrix 203 of predicted clinical phenotypes versus true phenotypes using a random forest on the 129 genes, illustrating the classification outcomes for patient clinical states, is depicted in FIG. 5.

The results show that the previously established stratification using over 900 genes, and employing the framework of the method for gene selection (around 129 genes identified), enables the reclassification of patients into homogeneous genomic subpopulations. Thus, these marker genes may be defined as biomarkers for patient stratification.

The method may be applied across various contexts, including diseases associated with the gut microbiota such as Inflammatory bowel disease (IBD), Ulcerative Colitis, Clostridium difficile infection, Colorectal Cancer for which relevant data are available.

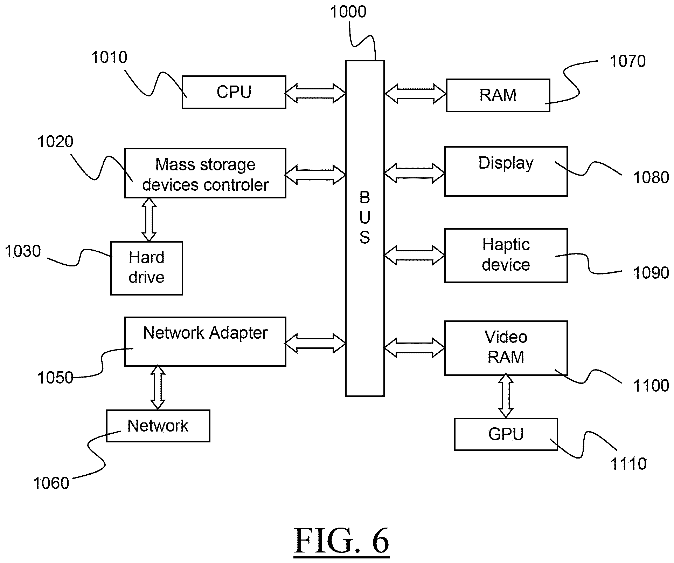

FIG. 6 shows an example of the system, wherein the system is a client computer system, e.g. a workstation of a user.

The client computer of the example comprises a central processing unit (CPU) 1010 connected to an internal communication BUS 1000, a random access memory (RAM) 1070 also connected to the BUS. The client computer is further provided with a graphical processing unit (GPU) 1110 which is associated with a video random access memory 1100 connected to the BUS. Video RAM 1100 is also known in the art as frame buffer. A mass storage device controller 1020 manages accesses to a mass memory device, such as hard drive 1030. Mass memory devices suitable for tangibly embodying computer program instructions and data include all forms of nonvolatile memory, including by way of example semiconductor memory devices, such as EPROM, EEPROM, and flash memory devices; magnetic disks such as internal hard disks and removable disks; magneto-optical disks. Any of the foregoing may be supplemented by, or incorporated in, specially designed ASICs (application-specific integrated circuits). A network adapter 1050 manages accesses to a network 1060. The client computer may also include a haptic device 1090 such as cursor control device, a keyboard or the like. A cursor control device is used in the client computer to permit the user to selectively position a cursor at any desired location on display 1080. In addition, the cursor control device allows the user to select various commands, and input control signals. The cursor control device includes a number of signal generation devices for input control signals to system. Typically, a cursor control device may be a mouse, the button of the mouse being used to generate the signals. Alternatively or additionally, the client computer system may comprise a sensitive pad, and/or a sensitive screen.

The computer program may comprise instructions executable by a computer, the instructions comprising means for causing the above system to perform the method. The program may be recordable on any data storage medium, including the memory of the system. The program may for example be implemented in digital electronic circuitry, or in computer hardware, firmware, software, or in combinations of them. The program may be implemented as an apparatus, for example a product tangibly embodied in a machine-readable storage device for execution by a programmable processor. Method steps may be performed by a programmable processor executing a program of instructions to perform functions of the method by operating on input data and generating output. The processor may thus be programmable and coupled to receive data and instructions from, and to transmit data and instructions to, a data storage system, at least one input device, and at least one output device. The application program may be implemented in a high-level procedural or object-oriented programming language, or in assembly or machine language if desired. In any case, the language may be a compiled or interpreted language. The program may be a full installation program or an update program. Application of the program on the system results in any case in instructions for performing the method. The computer program may alternatively be stored and executed on a server of a cloud computing environment, the server being in communication across a network with one or more clients. In such a case a processing unit executes the instructions comprised by the program, thereby causing the method to be performed on the cloud computing environment.

**Claims**

**1.** A computer-implemented method for genetic data processing, the method comprising:

- obtaining (S10) a dataset comprising microbial gene expression data for a plurality of patients;
- clustering (S20) the plurality of patients into a set of clusters based on the microbial gene expression data; and
- determining (S50) genes of which expression explains the clustering by:

    ○ selecting (S51) genes included in a set of metabolic pathways; and/or
    ○ identifying (S52) genes exhibiting significantly different expressions across the clusters.

2. The method of claim 1, wherein the clustering (S20) comprises:

    - performing (S21) several iterations of a Latent Dirichlet allocation, each iteration comprising assigning each patient to one of a plurality of topics;
    - determining (S22) a graph comprising nodes representing the plurality of patients and edges connecting pairs of nodes, the length of each edge connecting a pair of nodes being function of the number of occurrences of the patients represented by the connected nodes in the same topic over the several iterations of the latent Dirichlet allocation; and
    - performing (S23) a graph-based clustering of the determined graph.

3. The method of claim 2, wherein the graph is determined based on the Fruchterman-Reingold force-directed algorithm.

4. The method of claim 2 or 3, wherein the graph-based clustering is performed based on the Louvain clustering algorithm.

5. The method of any of claims 1 to 4, wherein the identifying (S52) of genes exhibiting significantly different expressions across the clusters comprises:

    - comparing the expression of each gene between each cluster; and
    - retaining genes with significantly different expressions between the clusters based on the results of the comparison.

6. The method of claim 5, wherein the comparison of the gene expression is performed based on a Kruskal-Wallis test computing a respective p-value for each gene, the retained genes having optionally p-values lower than 1 %.

7. The method of any of claims 1 to 6, further comprising selecting (S40) the set of metabolic pathways by extracting, from a database of existing metabolic pathways, the most significantly enriched metabolic pathways in terms of genes.

8. The method of claim 7, wherein the selecting (S40) of the set of metabolic pathways is performed based on a Fisher's exact test computing a respective p-value for each metabolic pathway, the selected metabolic pathways of the set having optionally p-values lower than 5 %.

9. The method of any of claims 1 to 8, further comprising filtering (S30), from a database of genes, genes of which relative expression is above a threshold, the genes being determined from the genes remaining after the filtering.

10. A computer implemented method of use of the clustering of claim 1, the method comprising:

    - obtaining microbial gene expression data for a patient, the microbial gene expression data including the expression of the genes determined in the method of claim 1;
    - determining the cluster to which the patient belongs based on the expression of the determined genes for the patient.

11. The method of claim 10, further comprising determining a closest patient among the patients of the determined cluster.

12. The method of claim 11, wherein the closest patient is determined using the Bray-Curtis distance.

13. A computer program comprising instructions which, when the program is executed by a computer, cause the computer to carry out the method of any of claims 1 to 9 and/or the method of any of claims 10 to 12.

14. A computer readable storage medium having recorded thereon a computer program of claim 13.

**15.** A system comprising a processor coupled to a memory, the memory having recorded thereon the computer program of claim 13.

Obtaining a dataset comprising microbial gene expression data for a plurality of patients — S10

Clustering the plurality of patients into a set of clusters based on the microbial gene expression data — S20

Performing several iterations of a Latent Dirichlet allocation — S21

Determining a graph — S22

Performing a graph-based clustering — S23

Filtering genes — S30

Selecting a set of metabolic pathways — S40

Determining genes of which expression explains the clustering — S50

Selecting genes included in the set of metabolic pathways — S51

Identifying genes exhibiting significantly different expressions across the clusters — S52

## FIG. 1

FIG. 2

FIG. 3

Distance individual based on distance metrics

FIG. 4

FIG. 5

1000

1010

CPU

1020

Mass storage
devices controler

1030

Hard
drive

1050

Network Adapter

Network

1060

B
U
S

1070

RAM

Display

1080

Haptic
device

1090

Video
RAM

1100

GPU

1110

## FIG. 6

Europäisches
Patentamt

European
Patent Office

Office européen
des brevets

# EUROPEAN SEARCH REPORT

**Application Number**

EP 24 30 6471

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | VÁZQUEZ-CASTELLANOS JORGE F ET AL: "Interplay between gut microbiota metabolism and inflammation in HIV infection", THE ISME JOURNAL, NATURE PUBLISHING GROUP UK, LONDON, vol. 12, no. 8, 23 May 2018 (2018-05-23), pages 1964-1976, XP036738036, ISSN: 1751-7362, DOI: 10.1038/S41396-018-0151-8 [retrieved on 2018-05-23] * the whole document, in particular the abstract, the sections "Material and methods" and "Results", figures 1-5 * | 1-15 | INV. G16B5/00 G16B25/10 G16B40/30 |
| X | WO 2022/226234 A1 (UNIV RUTGERS [US]) 27 October 2022 (2022-10-27) * abstract * * page 11 - page 36 * * figures 1,3-5 * | 1-15 | |
| A | OJALA TEIJA ET AL: "Current concepts, advances, and challenges in deciphering the human microbiota with metatranscriptomics", TRENDS IN GENETICS, ELSEVIER SCIENCE PUBLISHERS B.V. AMSTERDAM, NL, vol. 39, no. 9, 24 June 2023 (2023-06-24), pages 686-702, XP087372958, ISSN: 0168-9525, DOI: 10.1016/J.TIG.2023.05.004 [retrieved on 2023-06-24] * the whole document * | 1-15 | TECHNICAL FIELDS SEARCHED (IPC) G16B |

-/--

The present search report has been drawn up for all claims

| Place of search | Date of completion of the search | Examiner |
|---|---|---|
| Munich | 19 February 2025 | Hilbig, Matthias |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

&: member of the same patent family, corresponding document

EPO FORM 1503 03.82 (P04C01)

Europäisches
Patentamt

European
Patent Office

Office européen
des brevets

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | EETEMADI AMEEN ET AL: "The Computational Diet: A Review of Computational Methods Across Diet, Microbiome, and Health", FRONTIERS IN MICROBIOLOGY, vol. 11, 3 April 2020 (2020-04-03), XP055793499, DOI: 10.3389/fmicb.2020.00393 * the whole document * ----- | 1-15 | |

TECHNICAL FIELDS
SEARCHED     (IPC)

The present search report has been drawn up for all claims

| Place of search | Date of completion of the search | Examiner |
|---|---|---|
| Munich | 19 February 2025 | Hilbig, Matthias |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

page 2 of 2

## ANNEX TO THE EUROPEAN SEARCH REPORT
## ON EUROPEAN PATENT APPLICATION NO.

EP 24 30 6471

This annex lists the patent family members relating to the patent documents cited in the above-mentioned European search report.
The members are as contained in the European Patent Office EDP file on
The European Patent Office is in no way liable for these particulars which are merely given for the purpose of information.

19-02-2025

| Patent document cited in search report | Publication date | Patent family member(s) | Publication date |
|---|---|---|---|
| WO 2022226234 A1 | 27-10-2022 | EP 4326907 A1 | 28-02-2024 |
| | | IL 307845 A | 01-12-2023 |
| | | US 2024360522 A1 | 31-10-2024 |
| | | WO 2022226234 A1 | 27-10-2022 |

EPO FORM P0459

For more details about this annex : see Official Journal of the European Patent Office, No. 12/82

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **JIE et al.** The gut microbiome in atherosclerotic cardiovascular disease. *Nat Commun*, 2017, vol. 8, 845, https://doi.org/10.1038/s41467-017-00900-1